# EUROPEAN PATENT SPECIFICATION

(11) **EP 1 169 640 B1**
(45) Date of publication and mention of the grant of the patent: **15.06.2005**
(21) Application number: 00907857.7
(22) Date of filing: 09.03.2000
(51) Int. Cl.: G01N 33/50

(54) **METHOD FOR DETECTING CONDITIONS BY ANALYSIS OF AQUEOUS CONDENSATE FROM RESPIRATORY GASES**
DIAGNOSTISCHES VERFAHREN DURCH ANALYSE VON KONDENSIERUNG AUS ATMUNGS GAS
PROCEDE POUR DETECTER DES DESORDRES EN ANALYSANT LE CONDENSAT DES GAZ RESPIRATOIRES

(30) Priority: 09.03.1999 GB 9905259; 10.04.1999 GB 9908113
(43) Date of publication of application: 09.01.2002
(73) Proprietor: Osmetech PLC, Crewe, Cheshire CW1 1WZ (GB)
(72) Inventor: GRINDROD, David, Market Drayton, Shropshire TF1 3UB (GB); MARKELOV, Michael, Cleveland, OH 44131 (US)
(74) Representative: Lambert, Ian Robert
(86) International application number: PCT/GB2000/000859
(87) International publication number: WO 2000/054050

(56) References cited:
- WO-A-95/30765
- WO-A-95/33848
- WO-A-98/29563
- WO-A-98/39470
- WO-A-99/09407
- WO-A-99/09408
- GB-A- 2 203 553

## Description

This invention relates to a method for detecting the occurrence of a condition in a respiring subject, and to the detection and assessment of bodily fluids.

It is known from International Publication WO 95/33848 that microorganisms can be detected by detecting certain gaseous or volatile species emitted by the microorganisms, often as a result of metabolic process in the microorganism. International Publication WO98/39470 describes a method for detecting the occurrence of various conditions in a respiring subject in which certain characteristic gas phase species present in the emitted respiratory gases are detected using a gas sensing device. It has been found that, in practise, the results obtained with this method, in which emitted gases are directly detected by a gas sensing device, are less reliable and sensitive than is desirable. It is believed that one of the reasons for the difficulties encountered using the method of WO 98/39470 is the fact that exhaled respiratory gases contain a large amount of water vapour. Many gas sensing devices - including so-called "electronic noses", which are arrays of individual, often quite non-specific, gas sensors - are sensitive to water vapour, which can then have a masking effect.

An important area of application is in the detection of conditions in critically ill patients who are being artificially ventilated. In this instance, the above described problems are actually exacerbated since ventilators usually supply humidified air to the patient.

The present invention provides an improved method which overcomes the above described problems.

It is known that a number of diagnostic tests can be performed on cerebral spinal fluid (CSF), but these tests require a considerable period of time (typically ca. 24 hours) and are invalid if the sample of CSF is contaminated with serum.

Unfortunately, contamination with serum is not an uncommon event, because samples of CSF are collected by a somewhat risky and imprecise procedure. Furthermore, the procedure is at best uncomfortable, and frequently very painful, experience for the patient. Thus, it is highly undesirable for the patient as well as for the clinician that any contamination of the sample is detected a considerable time after extraction of the sample, since the whole sample collection procedure must then be repeated after a considerable delay (almost certainly on a different day). Furthermore, there is a risk attached to the collection procedure, because it is possible to rupture the membrane that protects the brain.

The present invention addresses the above described problems, and provides a quick test of the quality of a sample of CSF. The test can provide results whilst the patient is still physically prepared and available for the collection procedure, and, furthermore, whilst the patient is in still mentally prepared to endure further collection procedures.

According to a first aspect of the invention there is provided a method for detecting the occurrence of a condition in a respiring subject comprising the steps of:
obtaining an aqueous condensate from respiratory gases exhaled by the subject;
detecting certain species present in the aqueous condensate; and
correlating the presence of said species with the occurrence of the condition.

The species may be detected by detecting a gaseous or volatile species with a gas sensing device. The gaseous or volatile species may be the same as the species present in the aqueous condensate - in this instance, the species can be driven off, or simply evaporate from the condensate. Non-volatiles might be derivatised or otherwise treated in order to provide detectable gaseous or volatile species.

The gas sensing device may comprise a mass spectrometer. The gas sensing device may comprise a GC-MS.

The gas sensing device may comprise at least one gas sensor having a gas sensitive material of which an electrical or piezoelectrical property varies on exposure to a gas. The gas sensitive material may be semiconducting organic polymer.

The gas sensing device may comprise an array of gas sensors.

The aqueous condensate may be obtained from a ventilator.

The condition detected may be in interpulmonary infection, which may be pneumonia.

The species detected may be ammonia or an amine.

Methods in accordance with the invention will now be described with reference to the accompanying drawings in, which:
Figure 1 shows gas chromatographs obtained with a GC-MS system monitoring a m/z ratio of 17; and

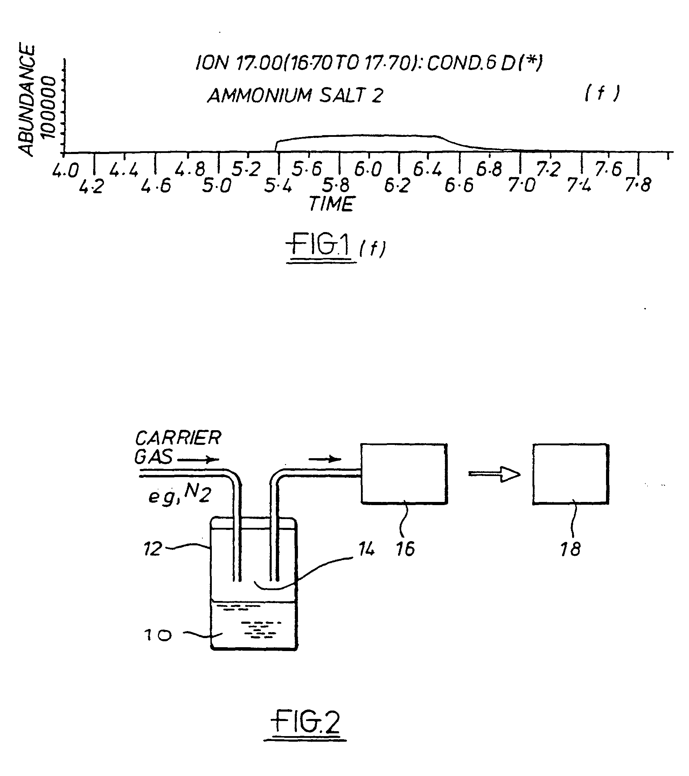

Figure 2 is a schematic diagram of a detection procedure for detecting CSF.

In one aspect, the invention comprises a method for detecting the occurrence of a condition in a respiring subject comprising the steps of:
obtaining an aqueous condensate from respiratory gases exhaled by the subject;
detecting certain species present in the aqueous condensate; and
correlating the presence of said species with the occurrence of the condition.

In this way, the water vapour in which is inevitably present in exhaled respiratory gases and which presents a major problem in the performance of the method of WO 98/39470 is actually used to advantage. It has been found that the condensate is rich in many important chemical species.

It should be noted that exhaled water vapour is substantially at body temperature and thus exhibits a marked tendency to condense upon contact with cooler surfaces. Schemes for collecting condensate are discussed below. It is possible that water droplets, carried in a subject's exhaled breath, might comprise some part of the collected condensate.

In a preferred embodiment, the species in the condensate are detected by detecting a gaseous or volatile species with a gas sensing device. Provided the species in the condensate is volatile, or is capable of being driven off, the condensate species itself may be detected in the gas phase. Alternatively, substantially non-volatile species might be derivatised or otherwise treated in order to provide a detectable or volatile species. Fatty acids, which are commonly occurring products of microorganism metabolism might be derivatised for this purpose.

Numerous classes of gas sensing device might be employed. Mass spectrometry - possibly as part of a GC-MC system-is one preferred technique, as described more fully below. So called "virtual sensors" might be employed. Other forms of gas chromatography might be employed.

In another preferred approach, the gas sensing device comprises at least one gas sensor having a gas sensitive material of which an electrical or piezoelectrical property varies on exposure to a gas.

The gas sensing device can comprises an array of semiconducting organic polymer gas sensors, the presence of gases being detected by monitoring variations in the dc resistances of said sensors. Such devices are manufactured, for example, by AromaScan plc, Crewe, UK. There are many semiconducting organic polymers suitable for such devices: examples include polypyrrole and polyindole and substituted variants thereof. It is a property of such polymer that the adsorption of certain (usually polar) gases causes measurable conductivity changes, and hence such gases may be detected by monitoring changes in the dc resistance of the polymer. Because each polymer typically exhibits quite broad band sensitivities, an array of gas sensors are employed. This permits selective identification of a wide range of gases by recognising the characteristic "fingerprint" of response across the array (see, for example, J V Hatfield, P Neaces, P J Hicks, K C Persaud and P Travers, Sensors and Acuators B, 18-19 (1994) 221-228). The output of the sensors can be analysed by analysis means, which correlates the output pattern of sensor response with the occurrence of certain conditions. The analysis means is usually a computer or microprocessor. A single such device can be used to recognise a number of conditions. The use of trained neural network is particularly advantageous in this regard.

It is also possible to interrogate semiconducting organic polymers by applying ac electrical signal thereto and monitoring an impedance quantity of the polymers, or variations in such an impedance quantity, as a function of the frequency of the applied ac electrical signal (see, for example, British Patent GB 2 203 553 and M E H Amrani. R M Dowdeswell, P A Payne and K C Persaud, Proceedings of Eurosensors X, Vol. 2, (1996) pp 665-668). Using this approach, a single semiconducting organic polymer sensor can provide information equivalent, or actually superior, to that provided by an array of such sensors interrogated by the dc resistance technique.

Other types of gas sensors which comprise a gas sensitive material might be employed, such as metal oxide semiconductor (MOS), quartz resonator or surface acoustic wave (SAW) devices. An array comprising a plurality of such devices would be utilised. Alternatively spectroscopic techniques such as infra-red spectroscopy might be employed.

An important but non-limiting area of application is in the detection of conditions in critically ill patients being artificially ventilated by a ventilator device. There are numerous means by which the aqueous condensate might be collected. For example, a conventional ventilator might be adapted to provide an easily accessible port from which the condensate can be extracted. A suitable run-off channel from the main ventilator air-way might be provided. A cold-finger or like device might be incorporated to encourage the formation of the condensate. Conventional ventilators are provided with receptacles which act to collect condensation: in practise it has been found to be convenient to extract a sample of aqueous condensate from this receptacle.

Measurements were performed on aqueous condensates obtained from the receptacles in ventilators being used to ventilate four patients. Two of the patients were presenting with pneumonia, the other two patients acting as the sources of reference condensates. Samples were prepared using ca. 80 µl of condensate and ca. 60 µl of sodium hydroxide.

The samples were evaporated into a GC-MS system set to monitor the m/z = 17 channel. This enable the production of ammonia to be detected. Measurements were also performed on a 1% solution of ammonium bromide.

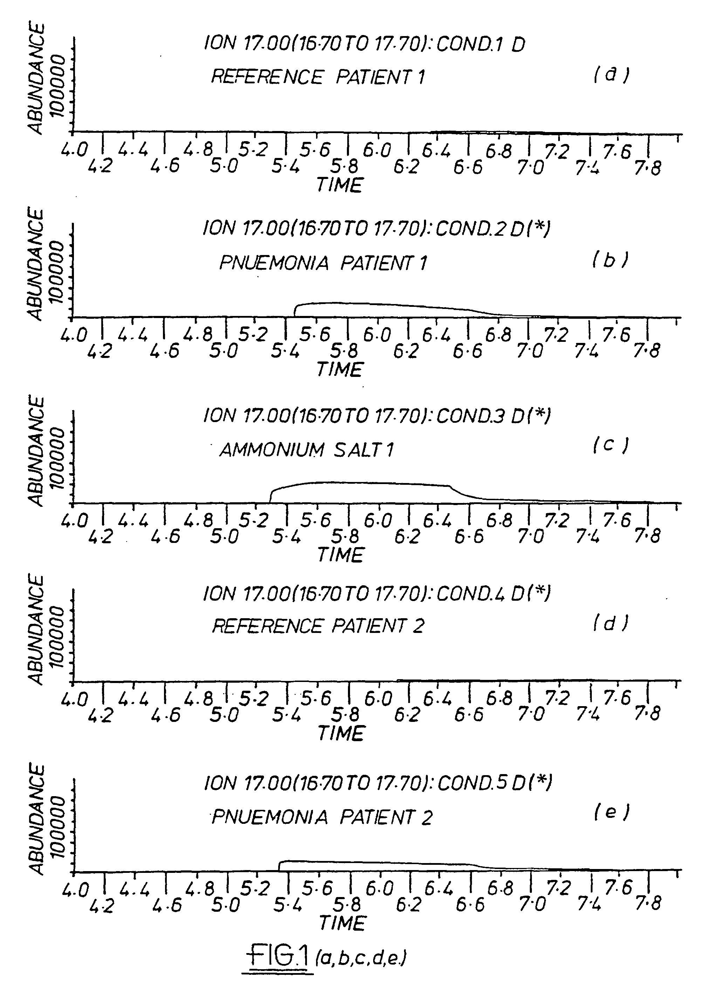

The resulting chromatographs are shown in Figure 1. Figures 1 (a) and 1 (d) corresponds the reference condensates, and show no significant signal in m/z = 17 channel. Figures 1 (b) and 1 (e) correspond to the condensates obtained from patients presenting with pneumonia. Significant ammonia production is observed. For comparison, Figures 1 (c) and 1 (f) show the ammonia signal generated from the ammonia bromide solution. It is estimated that this solution provides ca. 500 ppm of ammonia in the gas phase.

The sodium hydroxide raises the pH of the solution and thus drives out ammonia. It is possible that the sodium hydroxide might drive out small volatile amine species. Whilst it is believed that the species which is characteristic of pneumonia and present in the condensate is ammonia itself, it is possible that amine species might be present in addition to, or instead of, ammonia.

Further measurements were performed using an array of semiconducting organic polymer sensors (model A32S, AromaScan plc, Crewe, UK). A solution was prepared comprising 1 ml of water, 250 µl of aqueous condensate and ca. 100 µl of sodium hydroxide. Analysis of the gaseous headspace above the solution was performed using a "stripping" technique in which a carrier gas is passed across the surface of the solution and then sampled by the gas sensor array. The presence of ammonia was detected by the gas sensor array.

It is also possible to introduce gaseous or volatile species to the gas sensor array by a "sparging" technique in which a carrier gas is bubbled through the solution. Gaseous or volatile species are entrained in the carrier gas which is subsequently flowed across the array of sensors.

It is understood that the invention is not limited to the above example only. For example, other infectious conditions might be detected via species associated with microbial activity, such as short chain fatty acids and low molecular weight alcohols. Microorganisms will be present in the condensate, and it is possible to incubate these microorganisms under suitable conditions in order to increase the concentration of characteristic chemical species. Non-infectious conditions, such as intoxication by alcohol or other substances of abuse, might be detected. Depending on the nature of characteristic volatile "markers", it may be appropriate to add an additive, such as an acid or an alkali, in order to effect or enhance the release of characteristic volatile or volatiles. A portable collection device for the aqueous condensate can be provided, which a subject breathes into in order to provide a sample. A gas sensing device such as a gas sensing array might be integrated with such a device, or, alternatively, the condensate might be transported to the gas sensing device for analysis. In the case of a ventilator, a specially adapted ventilator might be provided in which the sample is collected from a position in closer proximity to the patient. Selective media might be employed in order to trap volatile species prior to their detection.

Instead of detecting gaseous and volatile species in the gas phase, it is possible to perform measurements in the liquid phase, i.e., directly on the condensate. Spectroscopic techniques such as infra-red spectroscopy might be used. Alternatively, a suitable test of the ELISA type might be contemplated. Other possibilities include analysis by electrochemical techniques, or by measuring electrical impedance, such as described in International Publication No. WO 98/46985.

In another aspect, the invention comprises a method for detecting bodily fluid comprising the steps of:
detecting gaseous or volatile species associated with a sample of liquid with a gas sensing device; and
correlating the presence of the said species with the presence of bodily fluid.

The method can be for assessing the purity of a sample of a bodily fluid, in which gaseous or volatile species associated with the sample of bodily fluid are detected with the gas sensing device, and the presence of said species is correlated with the purity of the sample of bodily fluid.

In a preferred embodiment, the bodily fluid is CSF, in which instance the presence of serum in samples of CSF can be detected. Alternatively, the bodily fluid might be blood, or serum.

As will become apparent below, it is not essential that the identity of the gaseous or volatile species is known, or that individual components of the gaseous "headspace" above the sample are identified.

The Figure shows a sample of CSF 10 stored within a container 12. Typically the sample of CSF 10 is obtained from a patient using known procedures and then detected shortly thereafter. Above the CSF 10 is a gaseous headspace 14 which contains certain gaseous or volatile species associated with the CSF 10. The headspace 14 is sampled by a gas sensing device 16 in an appropriate manner, such as by pumping. Data from the gas sensing device 16 are analysed by analysis means 18, typically a computer or a microprocessor.

In a preferred approach, the gas sensing device 16 comprises at least one gas sensor having a gas sensitive material of which an electrical or piezoelectrical property varies on exposure to a gas.

The gas sensing device 16 can comprise an array of semiconducting organic polymer gas sensors, the presence of gases being detected by monitoring variations in the dc resistances of said sensors. Such devices are manufactured, for example, by AromaScan plc, Crewe, UK. There are many semiconducting organic polymers suitable for such devices: examples include polypyrrole and polyindole and substituted variants thereof. It is a property of such polymer that the adsorption of certain (usually polar) gases causes measurable conductivity changes, and hence such gases may be detected by monitoring changes in the dc resistance of the polymer. Because each polymer typically exhibits quite broad band sensitivities, an array of gas sensors are employed. This permits selective identification of a wide range of gases by recognising the characteristic "fingerprint" of response across the array (see, for example, J V Hatfield, P Neaves, P J Hicks, K C Persaud and P Travers, Sensors and Actuators B, 18-19 ( 1994) 221-228). The output of the sensors can be analysed by the analysis means 18 which correlates the output pattern of sensor response with the state of the sample 10. The use of a trained neural network is particularly advantageous in this regard. Data reduction or cluster analysis techniques, such as Sammon Mapping, are also very useful, particularly for indicating differences between sampled headspaces. This is useful for indicating when a headspace - and hence the CSF sample - contains impurities.

It is also possible to interrogate semiconducting organic polymers by applying ac electrical signal thereto and monitoring an impedance quantity of the polymers, or variations in such an impedance quantity, as a function of the frequency of the applied ac electrical signal (see, for example, British Patent GB 2 203 553 and ME H Amrani. R M Dowdeswell, P A Payne and K C Persaud, Proceedings and Eurosensors X, Vol. 2, (1996) pp 665-668). Using this approach, a single semiconducting organic polymer sensors can provide information equivalent, or actually superior, to that provided by an array of such interrogated by the dc resistance technique.

Other types of gas sensors which comprise a gas sensitive material might be employed, such as metal oxide semiconductor (MOS), quartz resonator or surface acoustic wave (SAW) devices. An array comprising a plurality of such devices could be utilised. Alternatively spectroscopic techniques such as infra-red spectroscopy might be employed.

Further types of applicable gas sensing techniques include mass spectrometry - possibly as part of a GC-MC system-is one preferred technique, as described more fully below. So called "virtual sensors" might be employed. Other forms of gas chromatography might be employed.

Usually, the gaseous or volatile species detected are ordinarily present in the headspace above the sample. However, heating of the sample, acidification or treatment with alkali, or chemical conversion of any species present in the sample into a volatile species, or any other procedures to produce gaseous or volatile species from the sample, are within the scope of the invention. In the case of CSF and serum, it is believed that it is possible to differentiate between the two fluids because CSF is cleaner, serum being contaminated with many microorganisms, metabolic products thereof, and further waste products. In particular, it is believed that relatively high levels of ammonia are associated with serum. However, the invention should not be considered to be limited to the detection of this species alone, or limited by the explantion proposed above.

Typically, the sample of CSF is obtained from a patent using known procedures and then detected shortly thereafter. In this way, the success (or otherwise) of the procedure can be gauged very quickly. It may be possible to incorporate the gas sensing device into the equipment used to obtain the CSF sample. For example, a micro array of gas sensors might be incorporated into a syringe to produce a "smart syringe" capable of assessing the purity of the CSF sample.

## Claims

1. A method for detecting the occurrence of a condition in a respiring subject comprising the steps of:
obtaining an aqueous condensate from respiratory gases exhaled by the subject;
detecting certain species present in the aqueous condensate; and
correlating the presence of said species with the occurrence of the condition.

2. A method according to claim 1 in which the species are detected by detecting a gaseous or volatile species with a gas sensing device.

3. A method according to claim 2 in which the gas sensing device comprises a mass spectrometer.

4. A method according to claim 3 in which the gas sensing device comprises a GC-MS.

5. A method according to claim 2 in which the gas sensing device comprises at least one gas sensor having a gas sensitive material of which an electrical or piezoelectrical property varies on exposure to a gas.

6. A method according to claim 5 in which the gas sensitive material is semiconducting organic polymer.

7. A method according to claim 5 or claim 6 in which the gas sensing device comprises an array of gas sensors.

8. A method according to any previous claims in which the aqueous condensate is obtained from a ventilator.

9. A method according to any previous claims in which the condition detected is an intrapulmonary infection.

10. A method according to claim 9 in which the intrapulmonary infection is pneumonia.

11. A method according to any previous claims in which the species detected is ammonia or an amine.

## Patentansprüche

1. Verfahren zum Erfassen des Auftretens eines Zustands in einem atmenden Subjekt, welches die Schritte aufweist:
Erhalten eines wässrigen Kondensats aus von dem Subjekt ausgeatmeten Atemgasen;
Erfassen bestimmter in dem wässrigen Kondensat vorhandener Spezies; und
in Beziehung setzen der Anwesenheit dieser Spezies mit dem Auftreten des Zustands.

2. Verfahren nach Anspruch 1, bei dem die Spezies durch Erfassen einer gasförmigen oder flüchtigen Spezies mit einer Gasfühlvorrichtung erfasst werden.

3. Verfahren nach Anspruch 2, bei dem die Gasfühlvorrichtung ein Massenspektrometer aufweist.

4. Verfahren nach Anspruch 3, bei dem die Gasfühlvorrichtung ein GC-MS aufweist.

5. Verfahren nach Anspruch 2, bei dem die Gasfühlvorrichtung zumindest einen Gassensor mit einem gasempfindlichen Material, von dem sich eine elektrische oder piezoelektrische Eigenschaft verändert, wenn es einem Gas ausgesetzt wird, aufweist.

6. Verfahren nach Anspruch 5, bei dem das gasempfindliche Material halbleitendes organisches Polymer ist.

7. Verfahren nach Anspruch 5 oder Anspruch 6, bei dem die Gasfühlvorrichtung eine Anordnung von Gassensoren aufweist.

8. Verfahren nach einem der vorhergehenden Ansprüche, bei dem das wässrige Kondensat von einem Ventilator erhalten wird.

9. Verfahren nach einem der vorhergehenden Ansprüche, bei dem der erfasste Zustand eine intrapulmonale Infektion ist.

10. Verfahren nach Anspruch 9, bei dem die intrapulmonale Infektion Lungenentzündung ist.

11. Verfahren nach einem der vorhergehenden Ansprüche, bei dem die erfasste Spezies Ammoniak oder ein Amin ist.

## Revendications

1. Procédé destiné à détecter l'apparition d'un désordre dans un sujet respirant comprenant les étapes consistant à :
obtenir un condensat aqueux de gaz respiratoires expirés par le sujet ;
détecter certaines espèces présentes dans le condensat aqueux ; et
mettre en corrélation la présence desdites espèces avec l'apparition du désordre.

2. Procédé selon la revendication 1, dans lequel les espèces sont détectées en détectant des espèces gazeuses ou volatiles avec un dispositif de détection de gaz.

3. Procédé selon la revendication 2, dans lequel le dispositif de détection de gaz comprend un spectromètre de masse.

4. Procédé selon la revendication 3, dans lequel le dispositif de détection de gaz comprend un spectromètre de masse à chromatographie en phase gazeuse (CPG).

5. Procédé selon la revendication 2, dans lequel le dispositif de détection de gaz comprend au moins un capteur de gaz ayant un matériau sensible au gaz dont une propriété électrique ou piézoélectrique varie en fonction de l'exposition à un gaz.

6. Procédé selon la revendication 5, dans lequel le matériau sensible au gaz est un polymère organique semiconducteur.

7. Procédé selon la revendication 5 ou la revendication 6, dans lequel le dispositif de détection de gaz comprend un agencement de capteurs de gaz.

8. Procédé selon l'une quelconque des revendications précédentes, dans lequel le condensat aqueux est obtenu à partir d'un ventilateur.

9. Procédé selon l'une quelconque des revendications précédentes dans lequel le désordre détecté est une infection intra-pulmonaire.

10. Procédé selon la revendication 9, dans lequel l'infection intra-pulmonaire est une pneumonie.

11. Procédé selon l'une quelconque des revendications précédentes dans lequel les espèces détectées sont de l'ammoniac ou une amine.
